# EUROPEAN PATENT APPLICATION

(11) **EP 3 366 331 A1**
(43) Date of publication of application: **29.08.2018**
(21) Application number: 17158396.6
(22) Date of filing: 28.02.2017
(51) Int. Cl.: A61M 1/06, A61B 5/00, G01N 1/22

(54) **BREAST PUMP DEVICE COMPRISING A VOLATILE COMPONENT ANALYSIS SYSTEM**

(71) Applicant: Koninklijke Philips N.V., 5656 AE Eindhoven (NL)
(72) Inventor: AALDERS, Arnold, 5656 AE Eindhoven (NL)
(74) Representative: de Haan, Poul Erik

(57) **Abstract**

According to the invention, a breast pump device (1) is realized that is not only capable of realizing a breast milk extraction process, but that is also capable of providing relevant information about breast milk that is extracted, breast milk that has recently been extracted, a batch of stored breast milk, and/or the outside air. Milk-related air and/or outside air is analyzed by means of a volatile component analysis system (40) comprising at least one sensor (41) and a controller (42), wherein the controller (42) is configured and arranged to control an information device (50) for communicating the information that follows from the analysis to a user of the information device (50).

## Description

### FIELD OF THE INVENTION

The invention relates to a breast pump device for extracting breast milk from a human breast, comprising: (i) an expression kit comprising a breast-receiving funnel and a milk outlet, and (ii) a vacuum unit comprising a pump for realizing a pumping action on air in the breast-receiving funnel of the expression kit, particularly a pumping action involving evacuation of air from the breast-receiving funnel.

### BACKGROUND OF THE INVENTION

A breast pump device as mentioned in the opening paragraph is known from WO 2015/150225 A1, for example.

In general, breast pump devices are well known devices for extracting milk from a breast of a user, or two breasts simultaneously. A breast pump device may be used if the baby or infant is not itself able to extract milk from the breast, or if the mother is separated from the baby or infant, and the baby or infant is to be fed with breast milk by someone else. In other words, breast pump devices are used by mothers to express breast milk at a convenient time, to be stored for later consumption by their child. A breast pump device may also be helpful in a situation in which it is desired to stimulate and increase milk production in women with a low milk supply.

A breast pump device is typically operated with one or two expression kits. Among other things, an expression kit comprises a breast-receiving funnel for receiving a user's breast, which funnel may be equipped with pads or the like for massaging the breast in a certain way, and is designed for connection to a vacuum unit for realizing a pressure cycle in the breast-receiving funnel, by means of which milk expression from the breast is enabled. In practical cases, the vacuum unit comprises an electric vacuum pump. The fact is that by generating a pressure cycle, particularly a vacuum cycle, possibly accompanied by a certain way of massaging the breast, a simulation of a feeding action is obtained, which triggers the necessary let-down reflex in the user of the breast pump device.

There is a need for a breast pump device that is designed to not only be capable of extracting breast milk from a human breast, but also of providing information, particularly quality-related information, about the breast milk that is extracted by means of the device, during operation of the device and/or afterwards. Usually, the breast-receiving funnel and the milk outlet are part of a breast pump body of an expression kit, wherein furthermore a milk receptacle is provided that is connectable to the breast pump body, e.g. by screwing, at the position of the milk outlet. The milk receptacle serves for collecting breast milk that is extracted during operation of the breast pump device. Only in professional environments such as a hospital is it possible to have an accurate analysis performed of the composition of an extracted and collected batch of breast milk. Apart from that, a user of the breast pump device and/or a caregiver monitoring a user needs to rely on the look and the smell of a batch of breast milk in order to get an idea of the quality of the breast milk. This way of doing is not very accurate, and may leave the user and/or the caregiver in doubt as to the quality of the breast milk, and may even cause problems when insufficient or bad quality of breast milk goes unnoticed.

Quality of breast milk has several aspects. In a first aspect, quality of breast milk is related to the composition of the milk as far as the nutritive value thereof is concerned. It is known that at the start of lactation, so-called foremilk is expressed by the breast during a limited period, followed by so-called hindmilk, an important difference between the two types of breast milk relating to the milk fat concentration, wherein the hindmilk has a considerably higher milk fat content and a considerably lower water content than the foremilk. During a pumping session, it is practically impossible for a user and/or a caregiver of a user to visually distinguish the hindmilk from the foremilk and to recognize the period during which the composition of the milk changes, so that it is practically impossible for a user and/or a caregiver of a user to realize collection of milk in two batches, one batch being foremilk and another batch being hindmilk, if so desired. In a second aspect, quality of breast milk is related to the presence or absence of certain components in the milk. According to the state of the art, when a user and/or a caregiver of a user would like to know how the breast milk is influenced by the user's diet and/or if stress hormones are present in the breast milk, for example, it is only possible to obtain a vague impression of such relation from checking the color of the milk by looking at the milk and/or from checking the flavor of the milk by smelling the milk.

### SUMMARY OF THE INVENTION

It is an object of the invention to provide a breast pump device as referred to in the foregoing explanation of the need existing in the art, i.e. a breast pump device that is designed to not only be capable of extracting breast milk from a human breast, but also of providing information, particularly quality-related information, about the breast milk that is extracted by means of the device, during operation of the device and/or afterwards.

According to the invention, a breast pump device as defined in the opening paragraph is provided, being equipped with (iii) a volatile component analysis system that is designed to perform an analysis of air as present at a position in the breast pump device and/or of air as present at a position in the environment of the breast pump device, the volatile component analysis system comprising at least one sensor and a controller, wherein the at least one sensor is configured and arranged to detect at least one value of at least one volatile component in the air, and wherein the controller is configured and arranged to receive and process a detection signal from the at least one sensor representing the at least one detected value of the at least one volatile component, and (iv) an information device that is configured and arranged to provide a user of the information device with information relating to the at least one detected value of the at least one volatile component, the controller of the volatile component analysis system being configured and arranged to control the information device on the basis of the detection signal.

It follows from the foregoing definition that the breast pump device according to the invention is configured to perform a volatile component analysis of air as present at a position in the breast pump device and/or of air as present at a position in the environment of the breast pump device. In one aspect, the air to be analyzed may be milk-related air, i.e. air of a position in the breast pump device that is in fluid communication with a milk path of the expression kit to be followed by breast milk in the breast-receiving funnel and/or with a milk receptacle. Hence, milk-related air in the context of the invention can be air that is at a position near a quantity of breast milk that is collected during operation of the breast pump device or that has flown along such quantity of breast milk under the influence of a pumping action as performed by the pump of the vacuum unit during operation of the device. The fact is that air that is in contact with breast milk, either in a stationary condition or in a flowing condition, receives molecules evaporating from the milk. This is all the more the case when breast milk has just been extracted and is still relatively warm. One of the insights underlying the invention is that milk-related air contains volatile components originating from the milk and that this offers a possibility to detect at least one value of at least one volatile component of breast milk in air at an appropriate position in the breast pump device and to thereby obtain a reliable indication of an actual composition of the milk.

A notable advantage of the invention resides in the fact that a composition of the milk can be analyzed without needing to contact the milk, namely by "smelling" the milk by an appropriate analysis system, so that contamination of components such as sensors with milk is avoided, and actions of cleaning one or more components of the analysis system are not required. Based on the fact that evaporation effects of the milk take place and molecules of the milk are absorbed by air that is open to the milk, subjecting the air to analyzing actions yields a reliable indication of the composition of the milk. For example, the volatile component analysis system may be adapted to make a distinction between foremilk and hindmilk, in which case the information device may comprise a screen for providing a real-time indication of the type of milk to a user and/or a caregiver of a user, or may comprise a component for producing an audible sound at the moment it is found that the type of milk changes from foremilk to hindmilk. In any case, in this example, the volatile component analysis system may be designed to detect water vapor in milk-related air, particularly a concentration thereof, so as to be capable of recognizing the milk as being foremilk when the water vapor concentration is found to be above a predetermined threshold, and of recognizing the milk as being hindmilk when the water vapor concentration is found to be below the threshold. In another example, the volatile component analysis system may be adapted to detect the presence of one or more predetermined components and to provide appropriate indications to a user and/or a caregiver of a user on the basis thereof, wherein the indications may be actual indications of the presence or absence of the components concerned, or may be as general as "questionable quality", "good quality", "high level of stress hormones", "low level of stress hormones", "default flavor", "strong flavor" etc. On the basis of knowledge of information relating to the composition of the milk, either on a general level or a more specified level, the user is given the opportunity to adapt routines when it comes to factors that are known to influence the quality of breast milk, such as resting, eating, drinking, etc.

In case the volatile component analysis system is adapted to check whether breast milk may be qualified as being foremilk or hindmilk, a user of the breast pump device or a caregiver of a user, following indications about the type of milk provided through the information device, is enabled to collect the different types of milk in different receptacles if so desired. Alternatively, the breast pump device may be designed to offer a possibility of automatically separating the foremilk and the hindmilk. For example, two receptacles may be used with an expression kit, one receptacle being intended to collect foremilk and another receptacle being intended to collect hindmilk, and the controller of the volatile component analysis system may be adapted to ensure that milk that is detected as being foremilk is directed towards the first receptacle, and that milk that is detected as being hindmilk is directed towards the second receptacle.

On the basis of the fact that the breast pump device according to the invention comprises a volatile component analysis system and an information device, another possible application of the breast pump device is an application for measuring the quality of a batch of stored milk. When a container with stored milk is connected to the milk outlet of the expression kit instead of a receptacle for receiving extracted milk, the volatile component analysis system may be used to check the composition of the stored milk, especially for the purpose of determining whether the milk is still safe for consumption. In case an open air path is present between the expression kit and the vacuum unit, the components of the volatile component analysis system are preferably arranged in the vacuum unit, in which case a routine for performing an analysis involves activating the pump for creating a flow of air from the expression kit to the vacuum unit. Optionally, the breast pump device is equipped with a user interface for receiving input from a user or a caregiver of a user whether an analysis of a batch of stored milk is expected, and the controller may be programmed with a special algorithm to be followed in that case, resulting in detecting the presence and/or a quantitative characteristic such as the concentration of certain components that are known to be present in milk of bad quality.

In general, the at least one value of the at least one volatile component in the air to be analyzed may include at least one of the presence of the at least one volatile component and a quantitative characteristic of the at least one volatile component. For example, the at least one sensor of the volatile component analysis system may be configured and arranged to detect a concentration of water vapor in milk-related air, in which case the volatile component analysis system is suitable for determining whether milk can be denoted as being foremilk or hindmilk, as explained.

As suggested in the foregoing, the volatile component analysis system may be configured and arranged to perform the analysis of the air real-time during operation of the breast pump device, in which case it is advantageous for the controller of the volatile component analysis system to be configured and arranged to control the information device on the basis of a real-time detection signal during operation of the breast pump device.

In a practical embodiment of the breast pump device according to the invention, the at least one sensor of the volatile component analysis system is arranged at a position in the breast pump device where air that is displaced under the influence of the pump of the vacuum unit during operation of the breast pump device passes. In this respect, it is noted once again that in case an open air path is present between the expression kit and the vacuum unit, it is preferred for the components of the volatile component analysis system to be arranged in the vacuum unit, although basically it is also possible for one or more of those components to be arranged in the expression kit.

It is also possible for the volatile component analysis system to comprise at least one detection conduit accommodating at least one sensor, and for the at least one detection conduit to be configured and arranged so as to enable the pump of the vacuum unit to generate a flow of air towards and through the detection conduit. In other words, an embodiment of the breast pump device is possible in which air to be analyzed is directed towards a conduit that is especially provided for enabling the analysis function, wherein it is not necessary to use additional means for creating a flow of air as desired, but wherein it is possible to use the pump that is already available for that purpose instead.

Furthermore, in a practical embodiment of the breast pump device according to the invention, the volatile component analysis system comprises an electronic nose or a mass spectrometer.

It follows from the foregoing that the breast pump device according to the invention is suitable to be used for performing a volatile component analysis that is aimed at obtaining information about the composition of breast milk during a pumping session and/or right after a pumping session has taken place. Additionally or alternatively, the breast pump device may be designed to be capable of performing an analysis function of checking the quality of stored milk. On the basis of the presence of the volatile component analysis system in the breast pump device, the breast pump device may be designed so as to be capable of performing yet another analysis function if so desired, namely an analysis function aimed at providing an indication about the quality of the outside air (environmental air) at the location of the breast pump device, i.e. the location where a breast pumping action has taken place, is taking place or is supposed to take place. As the quality of the breast milk may be influenced by the quality of the outside air, a practical need may be fulfilled by means of the latter analysis function.

For the purpose of performing the outside air analysis function as mentioned in the preceding paragraph, use can be made of the fact that it is generally known for the vacuum unit of a breast pump device to be equipped with an air valve that is configured and arranged to release a vacuum in a continuous cycle during operation of the breast pump device. In particular, the volatile component analysis system may be designed to perform an analysis of outside air that is received in the vacuum unit through the air valve in the continuous cycle during operation of the breast pump device. In that case, it may be so that the volatile component analysis system comprises at least one sensor that is at a position for being in fluid communication with received outside air, wherein the at least one sensor is configured and arranged to detect at least one value of at least one component of received outside air, wherein the controller of the volatile component analysis system is configured and arranged to receive and process an outside air detection signal from the at least one sensor representing the at least one detected value of the at least one component of received outside air, and wherein the information device is configured and arranged to provide information relating to the at least one detected value of the at least one component of received outside air, the controller of the volatile component analysis system being configured and arranged to control the information device on the basis of the outside air detection signal.

For hygienic reasons, it is known for breast pump devices to be equipped with a milk leakage preventing arrangement that is configured and arranged such as to act as a barrier between the breast and the vacuum unit. According to a known option, such an arrangement may be provided as a non-permeable resilient silicone diaphragm, which needs to make a stroke for the purpose of creating a vacuum at the breast. The pump of the vacuum unit is operated to cause the diaphragm to flex, thereby expanding the air in the breast-receiving funnel and creating the required vacuum at the breast as a result thereof. When the vacuum at the pump side is released, the diaphragm will move back to its rest position. As the vacuum at the pump is indirectly causing the vacuum at the breast, the hygienic function of the diaphragm is implemented. This concept is well-established and has been used in the field of breast pump devices for many years.

Alternatively, an option of having an open air path between the expression kit and the vacuum unit exists, as suggested earlier. Normally, in breast pump devices designed according to that option, milk leakage preventing arrangements that are capable of blocking human breast milk while allowing air to pass are applied at a position between the expression kit and the vacuum unit. For example, such arrangements may comprise a breathable membrane that is gas-permeable and liquid-impermeable, and that has hydrophobic properties, as known from WO 2015/150225 A1. In particular, in a breast pump device in which an open air path is present between the expression kit and the vacuum unit, it is practical for the expression kit to comprise an air outlet and a milk leakage preventing arrangement that is associated with the air outlet and that constitutes a barrier to human breast milk while allowing air to pass, and for the vacuum unit to comprise an air inlet for receiving air from the air outlet of the expression kit. Furthermore, in such a case, it may be so that the pump of the vacuum unit is configured and arranged to suck air from the breast-receiving funnel of the expression kit, through the air outlet of the expression kit and the air inlet of the vacuum unit, and that the at least one sensor of the volatile component analysis system is arranged in the vacuum unit, at a position between the air inlet and the pump. Thus, the invention provides a possibility of having the at least one sensor of the volatile component analysis system, and possibly all components of the volatile component analysis system, in the vacuum unit, and still allowing realization of a proper analysis of milk-related air from the expression kit in view of the fact that it is possible for the vacuum unit to be in fluid communication with the expression kit when a suitable type of milk leakage preventing arrangement is applied, in which case air is allowed to travel from the expression kit all the way to the vacuum unit.

Various embodiments of a milk leakage preventing arrangement that is capable of blocking human breast milk while allowing air to pass exist are possible with the framework of the invention, including an embodiment in which the milk leakage preventing arrangement is hydrophobic and comprises a solid sheet of material, the sheet being provided with holes, an embodiment in which the milk leakage preventing arrangement is hydrophobic and comprises a porous membrane, and an embodiment in which the milk leakage preventing arrangement is hydrophobic and comprises a labyrinth.

The invention is particularly suitable to be applied in a context in which the breast pump device is of the electrical type, comprising an electric motor for driving the pump of the vacuum unit, and a conduit for interconnecting the expression kit and the vacuum unit. A practical example of a conduit as mentioned is a flexible hose. In the possible context of a breast pump device in which the expression kit comprises an air outlet and the vacuum unit comprises an air inlet, and in which it is intended to have a flow of air from the expression kit to the vacuum unit when the pump of the vacuum unit is operated, the conduit may particularly be arranged for establishing an open air path between the air outlet of the expression kit and the air inlet of the vacuum unit.

The information device may be designed in any way that is suitable for communicating information to a human being. For example, the information device may comprise a screen for displaying the information. Additionally or alternatively, the information device may be designed to provide a user of the information device with audible sounds. Furthermore, the information device may be separate from the other components of the breast pump device, i.e. the expression kit, the vacuum unit and the volatile component analysis system, and the controller of the volatile component analysis system may be configured and arranged to realize wireless transmission of the detection signal to the information device. In such a case, it is possible for a caregiver of a user of the breast pump device to check information that follows from a volatile component analysis at a remote location.

In practical embodiments of the breast pump device according to the invention, the controller of the volatile component analysis system is a microcontroller, which may be capable of retrieving preprogrammed information from a memory, wherein the preprogrammed information may be available in the form of a look-up table, for example, and/or which may be capable of following preprogrammed algorithms. In the context of the invention, the preprogrammed information and/or the preprogrammed algorithms are related to processing a signal received from the at least one sensor and controlling the information device in dependence on the signal.

The above-described and other aspects of the invention will be apparent from and elucidated with reference to the following detailed description of an embodiment of a breast pump device comprising an expression kit for application to a breast and a vacuum unit that is connectable to the expression kit through a hose and that serves for realizing a pressure cycle by means of which milk expression from the breast is enabled.

### BRIEF DESCRIPTION OF THE DRAWINGS

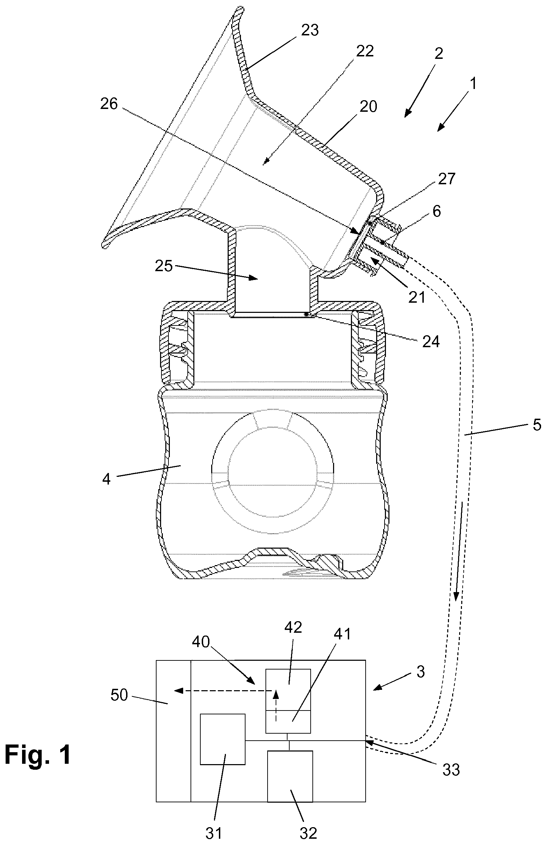

The invention will now be explained in greater detail with reference to figure 1, which diagrammatically shows a partially sectional view of a breast pump device according to a preferred embodiment of the invention, comprising an expression kit, a vacuum unit, a hose for interconnecting the expression kit and the vacuum unit, a volatile component analysis system that is arranged in the vacuum unit, and an information device, wherein components of the vacuum unit, components of the volatile component analysis system, and the information device are represented by blocks.

### DETAILED DESCRIPTION OF EMBODIMENTS

Figure 1 relates to a breast pump device 1 according to a preferred embodiment of the invention, comprising an expression kit 2 and a vacuum unit 3 for generating a pressure cycle during which vacuum is alternately created and released. The expression kit 2 comprises a breast pump body 20 and a milk receptacle 4 that is connectable to the breast pump body 20, e.g. by screwing, thereby closing a lower end of the breast pump body 20. In the shown example, the vacuum unit 3 is an electric vacuum unit and comprises an electric pump 31 and an air valve 32 for realizing an alternating vacuum during operation, i.e. during pumping sessions to be performed by means of the breast pump device 1. The electric pump 31, the air valve 32 and associated control means (not shown) for realizing proper operation of the electric pump 31 and the air valve 32 are designed to function in a manner that is well known in the field of breast pump devices. Therefore, further details of these components will not be further explained in the present text, and the same is applicable to other practical aspects of the vacuum unit 3 known per se, such as an electric connection of the pump 31 to a source of electric power, which may be the mains or a battery, for example.

In figure 1, the breast pump device 1 is shown in an assembled condition, in which the vacuum unit 3 is connected to the expression kit 2 through a hose 5. Such a configuration allows for a remote arrangement of the vacuum unit 3 with respect to the expression kit 2, so that the size of that part of the breast pump device 1 that is to be applied to a user's breast can be kept within reasonable limits. At one end, the hose 5 is connected to the expression kit 2 through a suitable connector 6 arranged at that particular end, which will hereinafter be referred to as expression kit connector 6. The hose 5 may be fixedly connected to the vacuum unit 3 at the other end thereof, but it also possible for the hose 5 to be releasably connected to the vacuum unit 3 through another suitable connector (not shown). It is to be noted that the breast pump device 1 can comprise two expression kits 2 for enabling a user of the breast pump device 1 to extract milk from two breasts at the same time, in which case the expression kits 2 can share a common vacuum unit 3.

The breast pump body 20 of the expression kit 2 has a first pressure chamber 21 and a second pressure chamber 22. The first pressure chamber 21 is configured for receiving the expression kit connector 6. The second pressure chamber 22 comprises a breast-receiving funnel 23, an aperture acting as a milk outlet 24, and a milk path 25 from the breast-receiving funnel 23 to the milk outlet 24. The breast-receiving funnel 23 is thus in fluid communication with the milk outlet 24 through the milk path 25. The breast-receiving funnel 23 can comprise a massage cushion or the like (not shown) for providing a soft and warm feel to the breast and/or imitating a baby's sucking action. In the shown example, the first pressure chamber 21 is located at a backside of the breast-receiving funnel 23. Within the framework of the invention, other locations of the first pressure chamber 21 are possible, including a location at the milk receptacle 4.

The breast pump device 1 according to the preferred embodiment of the invention as shown is of the type in which an open air path is established between the expression kit 2 and the vacuum unit 3. Hence, in the shown example, the expression kit 20 comprises an air outlet 26, and the vacuum unit 3 comprises an air inlet 33, wherein the air inlet 33 of the vacuum unit 3 serves for receiving air from the air outlet 26 of the expression kit 2, and wherein the pump 31 of the vacuum unit 3 serves for sucking air from the breast-receiving funnel 23 of the expression kit 2, through the air outlet 26 of the expression kit 2 and the air inlet 33 of the vacuum unit 3. Furthermore, in the shown example, the first pressure chamber 21 and the second pressure chamber 22 are separated at the position of a barrier portion 27 that is located in the breast pump body 20. The barrier portion 27 is designed to function as a milk leakage preventing arrangement at the position of the air outlet 26 of the expression kit 20 and may be realized as a solid sheet being provided with a number of holes having dimensions in the micrometer range, or as a porous membrane or a labyrinth, for example. Furthermore, at least at the position of the barrier portion 27, the material of the breast pump body 20 may have hydrophobic properties. In any case, it is intended for the barrier portion 27 to serve as an arrangement in the breast pump device 1 that is air-permeable and liquid-impermeable, at a position associated with the very position where air flows out of the expression kit 2 in the direction of the vacuum unit 3 during operation of the breast pump device 1. In that way, it is achieved that the barrier portion 27 serves for separating the first pressure chamber 21 from the milk path 25 in the second pressure chamber 22, thereby increasing the level of hygiene of the breast pump device 1 and preventing liquid from reaching the hose 5 and the vacuum unit 3, while allowing for air communication between the first pressure chamber 21 and the second pressure chamber 22, at least to such an extent that the breast milk expression functionality of the breast pump device 1 is not hampered, assuming that the barrier portion 27 provides a sufficiently low pneumatic restriction to the air flow. For example, a vacuum applied to the first pressure chamber 21 also causes vacuum in the second pressure chamber 22 since air can pass through the barrier portion 27, whereas water and/or breast milk in the second pressure chamber 22 are blocked. Thereby, the barrier portion 27 acts as a hygienic shield. Having a hydrophobic barrier portion 27 may further positively influence bacteria-related aspects of the breast pump device 1, such that bacteria transfer to the hose 5 and the vacuum unit 3 are prevented.

It is possible for the breast pump body 20 to be equipped with a splash guard (not shown) arranged in the second pressure chamber 22 for shielding the barrier portion 27 from droplets of breast milk. Thereby, such a splash guard can act as a first barrier which avoids that too much breast milk reaches the barrier portion 27. Droplets of breast milk which reach the barrier portion 27 anyway can clear off the barrier portion 27 automatically in case the barrier portion 27 is hydrophobic. Advantageously, the entire breast pump body 20 is made of one single material, preferably a clear plastic material having hydrophobic properties, such as polymethylpentene (PMP) or polypropylene (PP).

General operational aspects of the breast pump device 1 will now be mentioned. In the first place, a user and/or a caregiver of a user makes sure that the expression kit 2 and the vacuum unit 3 are properly connected to each other through the hose 5. Before the vacuum unit 3 is activated, the user and/or the caregiver furthermore needs to take care that the milk receptacle 4 is properly connected to the breast pump body 20, and that the breast to be subjected to a milk extraction process is properly inserted into the breast-receiving funnel 23 of the second pressure chamber 22. In that situation, a breast-receiving end of the second pressure chamber 22 is sealingly closed by the breast, whereas a lower end of the second pressure chamber 22 is sealingly closed by the milk receptacle 4. When, starting from that situation, the vacuum unit 3 is activated, a pressure cycle involving generation and release of vacuum is realized in the first pressure chamber 21, as a result of which the breast is subjected to forces which serve for simulating a feeding situation, as a result of which milk supply is induced from the breast, and during which it happens that air is sucked in the first pressure chamber 21 from the second pressure chamber 22 through the barrier portion 27. A desired pressure profile, i.e. a time-variable pressure, can be applied to the breast taking into account the pneumatic restriction of the barrier portion 27. The breast milk flows from the breast-receiving funnel 23 to the milk receptacle 4 through the milk path 25 and the milk outlet 24, under the influence of gravity and/or the pressure generated by the vacuum unit 3.

Preferably, the connection of the milk receptacle 4 to the lower end of the second pressure chamber 22 is airtight, although the invention also covers the possibility that leakage of air is allowed to a small extent at the position of the connection as mentioned. The fact is that the breast pump device 1 can still function properly when the pumping action to be realized by means of the pump 31 of the vacuum unit 3 is strong enough for invoking a flow of air from the expression kit 2 to the vacuum unit 3 as desired, in spite of some leakage of air as may occur in practice.

In the present embodiment of the invention, the breast pump device 1 is adapted to perform a function of providing a user and/or a caregiver of a user with information that is related to the quality of breast milk and that may be generated during a pumping session, right after a pumping session, or even in a separate session in case a user and/or a caregiver of a user wants to be informed about the quality of a batch of stored milk. To that end, the breast pump device 1 is equipped with a suitable volatile component analysis system 40, i.e. an analysis system that is designed to perform an analysis of air aimed at obtaining information about one or more volatile components as may be present in the air. In the context of the present embodiment of the breast pump device 1, the air to be subjected to an analysis is air that is in contact or has been in contact with breast milk, and the one or more volatile components are volatile components of the milk, i.e. molecules of the milk that have evaporated from the milk to the air. Furthermore, the breast pump device 1 is equipped with a information device 50, which may comprise a screen for displaying information derived from the volatile component analysis system 40 in written and/or graphic form to a user and/or a caregiver of a user, for example, and/or which may be separate, even be remote, from the other components of the breast pump device 1.

The volatile component analysis system 40 may comprise an electronic nose or a mass spectrometer, for example. In a more general sense, the volatile component analysis system 40 comprises at least one sensor 41 and a controller 42, wherein the at least one sensor 41 is configured and arranged to detect at least one value of at least one volatile component of breast milk in air that is present at an area that is open to an area for containing the milk or that is drawn from such an area, which air is referred to as milk-related air, and to transmit a breast milk detection signal representing the at least one detected value of the at least one volatile component of the milk to the controller 42. The controller 42 is configured and arranged to receive and process the breast milk detection signal from the at least one sensor 41, and to control the information device 50 on the basis of the breast milk detection signal. In figure 1, a volatile component analysis system 40 comprising one sensor 41 is illustrated, the one sensor 41 being arranged at a position in the vacuum unit 3, particularly a position between the air inlet 33 and the pump 31. Transmission of a detection signal from the sensor 41 to the controller 42 is diagrammatically indicated by means of a dashed arrow. Likewise, transmission of a control signal from the controller 42 to the information device 50 is diagrammatically indicated by means of a dashed arrow.

The type of information to be provided to a user and/or a caregiver of a user determines which type of detection needs to be performed during operation and which type of sensor 41 needs to be chosen. For example, the sensor 41 may be suitable to be used for detecting a concentration of water vapor in milk-related air. In such a case, it is possible to determine whether breast milk that is received and collected in the expression kit 4 is foremilk or hindmilk and to provide corresponding information through the information device 50. In another example, the sensor 41 may be suitable to be used for detecting the concentration of a component that is typically associated with the presence of stress hormones in the milk. In any case, by operating the pump 31 of the vacuum unit 3, milk-related air is drawn from the expression kit 2, and is forced to travel all the way down from the expression kit 2, passing the air outlet 26 of the expression kit 2 and the associated barrier portion 27, flowing through the hose 5 and passing the air inlet 33 of the vacuum unit 3, finally reaching the sensor 41 of the volatile component analysis system 40. By means of the controller 42 of the volatile component analysis system 40, information about milk as present in the expression kit 2 is generated by analyzing input as received from the sensor 41, and the information device 50 is controlled to present the information in such a way that it can be perceived by a human being. The direction in which the milk-related air flows through the hose 5 is diagrammatically indicated in figure 1 by means of an arrow.

It is possible for the volatile component analysis system 40 to be adapted to check whether one or more components indicating bad quality of breast milk are present in a batch of stored milk. In that case, the breast pump device 1 can be used as an analysis tool without performing a breast milk extracting process. A user or a caregiver of a user can put a container with a batch of stored milk at the position of the milk receptacle 4 and activate the breast pump device 1 so that the pump 31 of the vacuum unit 3 realizes a flow of milk-related air from the expression kit 2 to the vacuum unit 3, allowing the volatile component analysis system 40 as present in the vacuum unit 3 to analyze the air and control the information device 50 to provide an indication about the quality of the stored milk.

The breast pump device 1 may also be adapted to perform a function of providing information that is related to the quality of the outside air (environmental air) at the location where the breast pump device 1 is present, which may help a user and/or a caregiver of a user in deciding whether the location is suitable for safe use of the breast pump device 1, without any negative effects on the quality of the breast milk. To that end, the volatile component analysis system 40 may be designed to perform an analysis of outside air that is received in the vacuum unit 3 through the air valve 32 during operation and to control the information device 50 to provide information relating to one or more quality aspects of the air.

It follows from the foregoing that, according to the invention, a breast pump device 1 is realized that is not only capable of realizing a breast milk extraction process, but that is also capable of providing relevant information about breast milk that is extracted, breast milk that has recently been extracted, a batch of stored breast milk, and possibly also the outside air. Milk-related air and/or outside air is analyzed by means of a volatile component analysis system 40 comprising at least one sensor 41 and a controller 42, wherein the controller 42 is configured and arranged to control an information device 50 for communicating the information that follows from the analysis to a user of the information device 50.

It will be clear to a person skilled in the art that the scope of the invention is not limited to the examples discussed in the foregoing, but that several amendments and modifications thereof are possible without deviating from the scope of the invention as defined in the attached claims. It is intended that the invention be construed as including all such amendments and modifications insofar they come within the scope of the claims or the equivalents thereof. While the invention has been illustrated and described in detail in the figures and the description, such illustration and description are to be considered illustrative or exemplary only, and not restrictive. The invention is not limited to the disclosed embodiments. The drawings are schematic, wherein details that are not required for understanding the invention may have been omitted, and not necessarily to scale.

Variations to the disclosed embodiments can be understood and effected by a person skilled in the art in practicing the claimed invention, from a study of the figures, the description and the attached claims. In the claims, the word "comprising" does not exclude other steps or elements, and the indefinite article "a" or "an" does not exclude a plurality. Any reference signs in the claims should not be construed as limiting the scope of the invention.

Elements and aspects discussed for or in relation with a particular embodiment may be suitably combined with elements and aspects of other embodiments, unless explicitly stated otherwise. Thus, the mere fact that certain measures are recited in mutually different dependent claims does not indicate that a combination of these measures cannot be used to advantage.

The term "comprise" as used in this text will be understood by a person skilled in the art as covering the term "consist of'. Hence, the term "comprise" may in respect of an embodiment mean "consist of", but may in another embodiment mean "contain/include at least the defined species and optionally one or more other species".

The scope of the invention covers both the breast pump device 1 in an assembled condition as illustrated in figure 1 and the breast pump device 1 in a disassembled condition.

The expression kit 2 and the vacuum unit 3 may be positioned in any suitable configuration. According to one practical possibility, the expression kit 2 and the vacuum unit 3 are interconnected through a flexible hose 5, as is the case in the shown example, in which configuration it is possible to handle and manipulate the expression kit independently from the vacuum unit 3 to a considerable extent. According to another practical possibility, which is also covered by the invention, the vacuum unit 3 is attached to the breast-receiving funnel 23 of the expression kit 2.

## Claims

1. Breast pump device (1) for extracting breast milk from a human breast, comprising:
- an expression kit (2) comprising a breast-receiving funnel (23) and a milk outlet (24),
- a vacuum unit (3) comprising a pump (31) for realizing a pumping action on air in the breast-receiving funnel (23) of the expression kit (2), particularly a pumping action involving evacuation of air from the breast-receiving funnel (23),
- a volatile component analysis system (40) that is designed to perform an analysis of air as present at a position in the breast pump device (1) and/or of air as present at a position in the environment of the breast pump device (1), the volatile component analysis system (40) comprising at least one sensor (41) and a controller (42), wherein the at least one sensor (41) is configured and arranged to detect at least one value of at least one volatile component in the air, and wherein the controller (42) is configured and arranged to receive and process a detection signal from the at least one sensor representing the at least one detected value of the at least one volatile component, and
- an information device (50) that is configured and arranged to provide a user of the information device (50) with information relating to the at least one detected value of the at least one volatile component, the controller (42) of the volatile component analysis system (40) being configured and arranged to control the information device (50) on the basis of the detection signal.

2. Breast pump device (1) according to claim 1, wherein the at least one value of the at least one volatile component in the air includes at least one of the presence of the at least one volatile component and a quantitative characteristic of the at least one volatile component.

3. Breast pump device (1) according to claim 2, wherein the at least one sensor (41) of the volatile component analysis system (40) is configured and arranged to detect a concentration of water vapor in milk-related air of a position in the breast pump device (1) that is in fluid communication with a milk path (25) of the expression kit (2) to be followed by breast milk in the breast-receiving funnel (23) and/or with a milk receptacle (4).

4. Breast pump device (1) according to any of claims 1-3, wherein the volatile component analysis system (40) is configured and arranged to perform the analysis of the air real-time during operation of the breast pump device (1), the controller (42) of the volatile component analysis system (40) being configured and arranged to control the information device (50) on the basis of a real-time detection signal during operation of the breast pump device (1).

5. Breast pump device (1) according to any of claims 1-4, wherein the at least one sensor (41) of the volatile component analysis system (40) is arranged at a position in the breast pump device (1) where air that is displaced under the influence of the pump (31) of the vacuum unit (3) during operation of the breast pump device (1) passes.

6. Breast pump device (1) according to any of claims 1-5, wherein the volatile component analysis system (40) comprises at least one detection conduit accommodating at least one sensor (41), and wherein the at least one detection conduit is configured and arranged so as to enable the pump (31) of the vacuum unit (3) to generate a flow of air towards and through the detection conduit.

7. Breast pump device (1) according to any of claims 1-6, wherein the volatile component analysis system (40) comprises an electronic nose.

8. Breast pump device (1) according to any of claims 1-6, wherein the volatile component analysis system (40) comprises a mass spectrometer.

9. Breast pump device (1) according to any of claims 1-8, wherein the expression kit (2) comprises an air outlet (26) and a milk leakage preventing arrangement (27) that is associated with the air outlet (26) and that constitutes a barrier to human breast milk while allowing air to pass, wherein the vacuum unit (3) comprises an air inlet (33) for receiving air from the air outlet (26) of the expression kit (2), wherein the pump (31) of the vacuum unit (3) is configured and arranged to suck air from the breast-receiving funnel (23) of the expression kit (2), through the air outlet (26) of the expression kit (2) and the air inlet (33) of the vacuum unit (3), and wherein the at least one sensor (41) of the volatile component analysis system (40) is arranged in the vacuum unit (3), at a position between the air inlet (33) and the pump (31).

10. Breast pump device (1) according to claim 9, wherein the milk leakage preventing arrangement (27) that is associated with the air outlet (26) of the expression kit (2) is hydrophobic and comprises one of a solid sheet of material, the sheet being provided with holes, a porous membrane and a labyrinth.

11. Breast pump device (1) according to any of claims 1-10, comprising an electric motor (32) for driving the pump (31) of the vacuum unit (3), and a conduit (5) for interconnecting the expression kit (2) and the vacuum unit (3).

12. Breast pump device (1) according to claim 11 insofar as dependent on claim 9 or 10, wherein the conduit (5) is arranged to establish an open air path between the air outlet (26) of the expression kit (2) and the air inlet (33) of the vacuum unit (3).

13. Breast pump device (1) according to any of claims 1-12, wherein the information device (50) comprises a screen and is designed to display the information relating to the at least one detected value of the at least one volatile component on the screen.

14. Breast pump device (1) according to any of claims 1-13, wherein the information device (50) is separate from the other components of the breast pump device (1), i.e. the expression kit (2), the vacuum unit (3) and the volatile component analysis system (40), and wherein the controller (42) of the volatile component analysis system (40) is configured and arranged to realize wireless transmission of the detection signal to the information device (50).
